# EUROPÄISCHE PATENTSCHRIFT

(11) **EP 2 320 816 B1**
(45) Veröffentlichungstag und Bekanntmachung des Hinweises auf die Patenterteilung: **12.02.2014**
(21) Anmeldenummer: 09777876.5
(22) Anmeldetag: 07.08.2009
(51) Int. Cl.: A61B 17/72, A61B 17/80

(54) **KURZNAGEL ZUM VERSORGEN VON EPIPHYSEFRAKTUREN**
SHORT PIN FOR TAKING CARE OF EPIPHYSIS FRACTURES
CLOU COURT POUR TRAITER DES FRACTURES DE L'ÉPIPHYSE

(30) Priorität: 12.08.2008 DE 202008010922 U
(43) Veröffentlichungstag der Anmeldung: 18.05.2011
(73) Patentinhaber: Tantum AG, 24537 Neumünster (DE)
(72) Erfinder: JENSEN, Harm-Iven, 24214 Noer (DE); LÜCKERT, Ole, 24105 Kiel (DE)
(74) Vertreter: Stork Bamberger
(86) Internationale Anmeldenummer: PCT/EP2009/005895
(87) Internationale Veröffentlichungsnummer: WO 2010/017990

(56) Entgegenhaltungen:
- EP-A- 1 486 175
- WO-A-2004/039271
- WO-A-2007/035440
- DE-U1- 9 106 152

## Beschreibung

Die Erfindung betrifft einen Kurznagel zum Versorgen von gelenknahen Epihysefrakturen eines Epiphyse und Diaphyse aufweisenden Röhrenknochens, umfassend einen an die Dimension der Frakturepiphyse angepassten, zum Einbringen mit lateralem, gelenkfernem Zugangsweg eingerichteten zu implantierenden Nagelstützkörper und, im Zustand der Frakturversorgung, wenigstens einen den Nagelstützkörper mit diesem in gekreuzter Anordnung durch Querdurchbohrung durchsetzenden Befestigungsstift, der mit winkelfester Anbringung an dem Nagelstützkörper und mit Sicherung gegen Bewegung längs der Stiftachse vorgesehen ist, wobei der Nagelstützkörper so dimensioniert und eingerichtet ist, dass er in der Frakturepiphyse in Schräglage zu liegen kommt und sein an der lateralen Einbringungsseite zu liegen kommendes Ende eine Diaphyseschräge aufweist, die am Außenumfang der Diaphyse in einem Diaphysebereich wenigstens nahezu bündig abschließt, der an die sich zwischen der Frakturepiphyse und der Diaphyse erstreckende Metaphyse anschließt.

Mit dem Kurznagel wird ein intramedulläres Implantat zur Verfügung gestellt, das die vorhandene Knochenstruktur stützt und erhält. Im wesentlichen Unterschied zu sich lang erstreckenden Knochennägeln, zum Beispiel zum Versorgen von Oberarmfrakturen, die antegrad gelenknah eingebracht und in der Markhöhle der Diaphyse verankert werden (vgl. zum Beispiel US 5,472,444), erlaubt der Kurznagel eine gering invasive Operationstechnik, die die vorhandene Knochenstruktur der Epiphyse auch bei problematischen Frakturen stützt und erhält. Das Gelenk wird nicht geöffnet. Der Zugangsweg perkutan von lateral reduziert das Gewebetrauma. Die Stabilisierung von Epiphysefragmenten erfolgt durch die gekreuzten Befestigungsstifte. Ein solches System ist speziell für subkapitale und pertuberkuläre Humerusfrakturen geeignet.

Die Operationstechnik mit Kurznagel ist auch von Operationstechniken mit Plattensystemen zu unterscheiden. Zum Beispiel ist aus US 5,693,055 eine Plattenanordnung mit einer auf Kompression beanspruchten Knochenschraube bekannt. Zwar wird die Knochenschraube in Schräganordnung lateral durch die Diaphyse eingebracht. Die Schraube ist jedoch eine Spannschraube, die als solche keinen Stützkörper für Verriegelungs-/Be-festigungsstifte bildet, indem sie gegen eine in dem Bereich von Diaphyse und Metaphyse anzubringende Platte zum Aneinanderziehen von Knochenfragmenten zu spannen ist. Ein Teil der Platte ist in eine besondere Aufbohrung der Diaphyse einzubringen. Die unter Kompression stehende Schraube kann Mehrfragmentfrakturen nicht erfassen und stabilisieren. Andere bekannte Systeme mit an den Knochen im gelenknahen Bereich angesetzten Platten, die als Kraftträger für Verriegelungsschrauben dienen (z. B. DE-T2-602 20 175), sind zur Versorgung nur bedingt und eingeschränkt nutzbar. Insbesondere besteht das Erfordernis gelenknaher Operation. Die Operationstechnik ist besonders invasiv und führt zu Traumatisierung. Mit den Plattensystemen können kurze Fraktursegmente nicht erfasst werden.

Ein gattungsgemäßer Kurznagel zum Versorgen von Kopffrakturen eines Knochens wird in WO-A2-2004039271 offenbart. Wenngleich der dort beschriebene Kurznagel die Operationstechnik und die Behandlung von insbesondere problematischen Mehrfragmentfrakturen wesentlich verbessert, so hat sich herausgestellt, dass Befestigungselemente im Bereich des chirurgischen Halses bei einigen Anwendungen für eine zufriedenstellende Stabilisierung des Kurznagels nicht ausreichen. Hier soll die Erfindung Abhilfe schaffen.

Danach liegt der Erfindung die Zielsetzung zugrunde, die Stabilisierung des Kurznagels zu verbessern und die Operationsindikationen zu erweitern.

Das Ziel wird in Verbindung mit den Merkmalen des Knochen-Kurznagels der eingangsgenannten Art dadurch erreicht, dass der Nagelstützkörper zweiteilig mit einem die wenigstens eine Querdurchbohrungen für den Befestigungsstift aufweisenden Hauptkörper und einem die Diaphyseschräge aufweisenden Stummelkörper ausgebildet ist, der mit dem Hauptkörper entsprechendem Stützquerschnitt versehen und fester Bestandteil eines Diaphyseankers ist, der mittels des Stummelkörpers und eines den Hauptkörper und den Stummelkörper steif verbindenden Verbindungsmittels an den Hauptkörper lösbar ansetzbar ist und der mit einem Verbindungs-Flachsteg ausgebildet ist, der in schräger Ausrichtung mit der Diaphyseschräge mit dieser lateral flächig abschließt, von der Frakturepiphyse wegweisend flach an der Diaphyse zur Anlage kommt und mit einem Verankerungsmittel ausgestattet ist, durch das der Flachsteg in flacher Anlage an der Diaphyse festsetzbar ist.

Erfindungsgemäß erreicht man, dass der implantierte Kurznagel besonders wirksam und zuverlässig stabilisiert ist. Der Stützkörper des Kurznagels ist mit dem Diaphyseanker derart ausgebildet und eingerichtet, dass ohne Auswirkung auf die Nagellänge und damit ohne Beeinträchtigung der Schräglage in der Frakturepiphyse die stabile Stützfunktion gewährleistet und ein Ausbrechen zum Knochenschaft hin zuverlässig vermieden wird. Wesentlich ist, dass der Stummel- oder Ansatzkörper des Stützkörpers mit der Diaphyseschräge Bestandteil des Diaphyseankers ist, so dass der Stützkörper mit der Diaphyseschräge an dem Diaphyseanker bzw. an dessem Verbindungs-Flachsteg bündig abschließt. Mit dem Verbindungs-Flachsteg erreicht man, dass die Diaphyseschräge am Außenumfang der Diaphyse wenigstens nahezu bündig abschließt.

Ein Befestigungsteil des Verbindungs-Flachstegs ist durch ein flachförmiges Ansatz- oder Verlängerungsstück nach Art einer flachen Fahne oder Zunge bestimmt. Damit trägt der Verbindungs-Flachsteg am Diaphyseumfang nicht auf. Obwohl die stabile, nur in den Bereich der Metaphyse oder des chirurgischen Halses hineinreichende Schräglage des Kurznagels in der Frakturepiphyse erreicht wird, gelingt es, die Verankerung in gelenkfernem Bereich der Diaphyse und dort mit einfach anbringbarem, Traumatisierung reduzierendem Verankerungsmittel, zweckmäßig mit einer in die Diaphyse eingeschraubten Verbindungsschraube herzustellen. Kraftverhältnisse zur stützenden und stabilisierenden Befestigung des Stützkörpers sind besonders günstig, da der Verbindungs-Flachsteg des Diaphyseankers lateral außen unter flachem stumpfem Winkel in den Stummelkörper des Stützkörpers übergeht. Der Verbindungs-Flachsteg bleibt unter Gewährleistung von Stütz- und Befestigungsfunktion mit der in der Diaphyse unter das Schrägende des Stützkörpers verlegten Verankerung besonders flach und relativ kleinflächig. Vorzugsweise wird der Verbindungs-Flachsteg so kurz gehalten, dass die Verankerung in der Nähe des Stützkörper-Schrägendes unter diesem zu liegen kommt. Aufgrund der erfindungsgemäßen Gestaltung wird ein besonderer Eingriff des Diaphyseankers in die Diaphyse vermieden.

Im Ganzen erzielt man ein Kurznagel-Implantat, dessen als intramedullärer Kraftträger dienender Stützkörper in besonderem Maße stabilisiert ist. Es resultiert eine optimale Stabilisierung der Kopffragmente mittels der Befestigungsstifte. Die stabile Frakturversorgung, insbesondere auch bei Mehrfragmentfrakturen, erreicht man sowohl für vitale, als auch für porotische Knochenstrukturen. Vorteile gering invasiver Operationstechnik, damit insbesondere Erhaltung des Knochenkopfes, Schonung von Bändern und Sehnen als Voraussetzung für gute Repositionsergebnisse sowie Vermeiden von Traumatisierung während der Versorgung kommen zur Geltung.

Die erfindungsgemäße Verankerung wird besonders vorteilhaft in Kombination mit einem am Knocheneingriffsende des Stützkörpers ausgebildeten Außengewinde vorgesehen. Man erreicht, dass der Stützkörper an beiden äußeren Enden fixiert ist.

Zweckmäßig besteht das den Hauptkörper und den Stummelkörper des Stützkörpers verbindende Verbindungsmittel in einer Schraubverbindung. Die Verbindung ist steif in dem Sinne, dass der Hauptkörper und der Stummelkörper auf Stoß und gegen relatives Verschieben und Drehen zueinander gesichert aneinandergezogen sind.

Das den Hauptkörper und den Stummelkörper des Stützkörpers verbindende Verbindungsmittel kann mit einer Drehsicherung eingerichtet sein, die eine relative axiale Drehung zwischen den beiden Körpern in einstellbarer Drehposition form- und/oder kraftschlüssig sperrt.

Eine vorteilhafte Ausgestaltung besteht auch darin, dass das Verankerungsmittel des Diaphyseankers mit einem Rastmittel eingerichtet ist, das wenigstens zwei jeweils eine Höhenlage des Diaphyseankers an der Diaphyse bestimmende Rastpositionen ausbildet, die wählbar sind und den Diaphyseanker in festgesetztem Zustand gegen von der Epiphyse längs der Diaphyse weg gerichtete Bewegung sichern.

In vorteilhafter Gestaltung wird der Stummelkörper mit an der Diaphyseschräge offenem, einen Durchgang bildendem Hohlraum ausgebildet, und die den Diaphyseanker steif mit dem Hauptkörper des Stützkörpers verbindende Schraubverbindung umfasst eine von lateral setzbare Verbindungsschraube mit einem Kopfbereich, der in dem Hohlraum zumindest im Wesentlichen versenkt zu liegen kommt. Eine besonders einfache Verankerung des Diaphyseankers erreicht man dadurch, dass das Verankerungsmittel ein entsprechend der Diaphyse sich erstreckendes, an seinem Verbindungs-Flachsteg ausgebildetes Langloch und eine durch dieses hindurchsetzbare Verankerungsschraube umfasst. Zweckmäßig besteht eine Gestaltung des genannten Rastmittels darin, dass das Langloch als Rastschablone zur höhenveränderlichen Rastaufnahme des Kopfes der Verankerungsschraube ausgebildet wird.

Zweckmäßig werden der Stummelkörper des Stützkörpers und der Verbindungs-Flachsteg kleinbauend so dimensioniert und ausgebildet, dass der Verbindungs-Flachsteg bündig an den Stummelkörper anschließt, wobei er auch zur Seite der Metaphyse mit dem Stummelkörper wenigstens nahezu randlos abschließt.

Eine Besonderheit des erfindungsgemäßen Stützkörpers besteht darin, dass das laterale Ende des Stützkörpers in Form des Stummelkörpers als Basis oder Fuß des Diaphyseankers ausgebildet ist. Zweckmäßig ist der Stummelkörper einstückiger Bestandteil des Diaphyseankers und zusammen mit diesem geformt. Der zweiteilig bauende, den Diaphyseanker aufweisende Stützkörper lässt sich als Einheit für das Implantat konfektionieren und anbieten.

Der erfindungsgemäße Kurznagel wird zweckmäßig in Form einer Sachgesamtheit, die eine Verkaufseinheit bildet, angeboten. Die Sachgesamtheit zum Herstellen eines Kurznagel-Implantates, insbesondere für proximale Humerusfrakturen, umfasst die Teile des erfindungsgemäßen Kurznagels bzw. Stützkörpers, nämlich wenigstens in Einzahl jeweils den Hauptkörper, den Diaphyseanker mit Stummelkörper, dafür das Verbindungsmittel und das Verankerungsmittel sowie wenigstens einen Satz von Befestigungsstiftten.

Unteransprüche sind auf die genannten und noch andere zweckmäßige und vorteilhafte Ausgestaltungen gerichtet. Besonders zweckmäßig und vorteilhafte Ausbildungsformen oder -möglichkeiten der Erfindung werden anhand der folgenden Beschreibung der in der schematischen Zeichnung dargestellten Ausführungsbeispiele näher beschrieben. Es zeigen
- Fig. 1: in Explosionsdarstellung und seitlicher Vorderansicht einen erfindungsgemäßen Kurznagel zum Versorgen einer Mehrfragmentfraktion des Humerus,
- Fig. 2: in Ansicht von dorsal den aus den Teilen der Fig. 1 zusammengefügten erfindungsgemäßen Kurznagel als Implantat im Humerus und
- Fig. 3 und 4: den Knochennagel gemäß Fig. 1 und 2 in Ansicht von dorsal bzw. lateral.

Der im Ausführungsbeispiel dargestellte Kurznagel 1 ist als Implantat für subkapitale und pertuberkuläre Frakturen des Humerus 7 eingerichtet. Der Kurznagel 1 ist für die genannten Humerusfrakturen besonders geeignet. Allgemein ist der erfindungsgemäße Kurznagel mit angepasster Dimensionierung und Auslegung insbesondere zum Versorgen von Mehrfragmentfrakturen der Epiphysen von Röhrenknochen geeignet, wie zum Beispiel auch von Femur, Tibia, Fibula, Radius und Ulna. Der erfindungsgemäß gestaltete Kurznagel kann aber auch für andere Knochen mit gelenknahen Frakturen, zum Beispiel für Frakturen des Calcaneus verwendet werden..

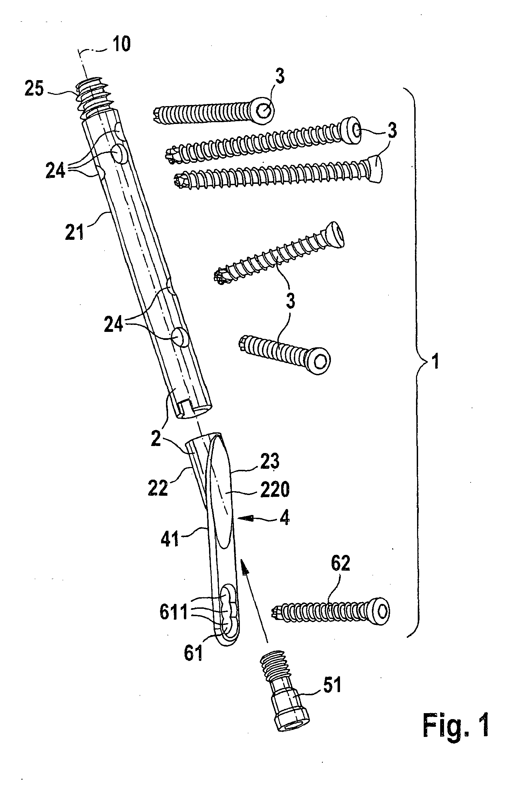

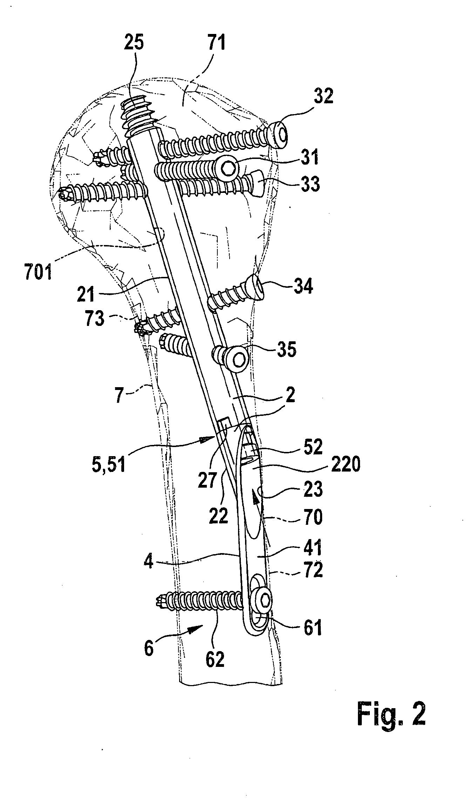

Aus Fig. 2 gehen Lage, Aufbau und Bestückung des im Humerus 7 implantierten Kurznagels 1 hervor. Bruchlinien werden im Humeruskopf (Epiphyse des Humerus) 71 nicht näher dargestellt. Einzelne Bauteile des Kurznagels 1 sind aus Fig. 1 ersichtlich. Der Kurznagel 1 weist einen Stützkörper 2, Befestigungsstifte 3, einen Diaphyseanker 4 sowie ein Verbindungsmittel 5 und ein Verankerungsmittel 6 auf.

Der Stützkörper 2 ist zweiteilig ausgebildet. In Form eines Stabes oder Nagels mit glattem Schaft hat er die Funktion eines Kraftträgers in Verbindung mit den ihn durchsetzenden Befestigungsstiften 3. Er setzt sich aus einem langgestreckten geraden Hauptkörper 21 und einem demgegenüber kurzstückigen Stummel- oder Fußkörper 22 zusammen. Im Ausführungsbeispiel weisen die beiden Körper 21, 22 übereinstimmenden und gleichbleibenden Kreisquerschnitt auf, und sie sind als Hohlzylinder ausgeführt. Mittels des Verbindungsmittels 5 sind die beiden Körper 21, 22 in gerader Ausrichtung im Stoßsitz an zugewandten Enden mit zur geraden Nagelachse 10 senkrechten Stoßflächen fest und steif aneinandergefügt. Die Verbindung ist derart, dass die beiden Körper 21, 22 relativ zueinander drehfest und axial unverschieblich aneinandersitzen.

Genauer betrachtet, weist der Stummelkörper 22 die Form eines schräg abgeschnittenen hohlen Kreiszylinderstücks auf. Der Schrägschnitt bzw. die Schrägfläche liegen unter stumpfem weitem Winkel 26 zur Längsachse 10 des Kurznagels 1. Die Schrägfläche bildet eine bündig mit der Diaphyseoberfläche abschließende Diaphyseschräge 23 an dem Stützkörper-Ende, das an der lateralen Einbringungsseite des Kurznagels 1 zu liegen kommt.

Der Diaphyseanker 4 ist als einstückiges oder einteiliges, jedenfalls eine feste Körpereinheit bildendes Bauteil gestaltet bzw. gearbeitet. Bestandteile sind ein Verbindungs-Flachsteg 41 und der Stummelkörper 22. Der Flachsteg 41 umfasst ein flaches längliches Flachstück nach Art einer Befestigungsfahne oder -zunge, das an dem Stummelkörper 22 ausschließlich nach distal, also von dem Humeruskopf (Frakturepiphyse) 71 weg gerichtet absteht. Der Diaphyseanker 4 ist derart gestaltet, dass die Diaphyseschräge 23 in die laterale Flachfläche des Flachstegs 41 übergeht bzw. damit zusammenfällt. Die Diaphyseschräge 23 ist an der am Umfang der Diaphyse 72 sich länglich erstreckenden Flachfläche des Diaphyseankers 4 offen. Der Stummelkörper 23 geht randlos bündig in die Flachfläche und damit in den Flachsteg 41 über. Dieser Übergang ist proximal bündig und randlos, so dass der Diaphyseanker 4 mit dem Flachsteg 41 ausschließlich distal zur formschlüssigen Flachanlage an die im Wesentlichen gerade Wandung der Diaphyse 72 kommt. Zudem ist der Stummelkörper 22 so kurzstückig ausgebildet, dass der Flachsteg 41 bzw. die der Diaphyseschräge 23 entsprechende Schrägöffnung proximal, also zur Seite der Frakturmetaphyse 73 mit dem Stummelkörper 22 überstandsfrei abschließt. Anders ausgedrückt, schließt die den Stoßsitz herstellende Anschlussfläche des Stummelkörpers 22 proximal mit der Flachfläche des Diaphyseankers 4 ab und steht darüber proximal nicht hervor.

Mit dem beschriebenen kurzen Stummelkörper 22 des Diaphyseankers 4 wird in besonderem Maß erreicht, dass der Anschluss des Diaphyseankers 4 an den Hauptkörper 21 des Stützkörpers 2 so weit wie möglich nach lateral, jedoch in der Diaphyse 72 versenkt, gelegt ist.

Das Verbindungsmittel 5 ist als Schraubverbindung 51 mit einer Verbindungsschraube 52 ausgebildet. Diese greift in ein Innengewinde innen am distalen Ende des Hauptkörpers 21 ein und kommt mit ihrem Kopf in dem Durchgang oder Hohlraum 220 des Stummelkörpers 22 in versenkter Anordnung zu liegen. Die mittels der Schraubverbindung 51 auf Stoß gegeneinandergezogenen Anschlussenden der beiden Körper 21, 22 bilden im Kraftschluss eine Drehsicherung, die in einstellbarer Position die relative axiale Drehung zwischen den beiden Körpern 21, 22 sperrt. Die Feinjustierung der relativen Drehposition der Körper 21, 22 zueinander ist von Bedeutung, um eine weitgehend großflächige, flache Anlage des Flachstegs 41 an der Diaphyse 72 herzustellen. Die einstellbare Drehposition kann zum Beispiel auch durch eine feinjustierende Rastverbindung mit ineinandergreifenden Rastscheiben od. dgl. eingerichtet werden.

Der Hauptkörper 21 des Stützkörpers 2 weist an seiner lateralen Stirnseite eine Nut 27 zum Anschluss eines herkömmlichen Adapters für Zielgerät und Drehwerkzeug zum Ausrichten und Einbringen des Nagel-Hauptkörpers 21 auf.

Der Diaphyseanker 4 wird mittels des Verbindungs-Flachstegs 41 distal unterhalb der Endschräge 23 des Kurznagels 2 in der Nähe dieses Nagelendes an der Diaphyse 72 befestigt. Diese Verankerungsverbindung umfasst als Verankerungsmittel 6 eine Verankerungsschraube 62 mit Vollgewinde sowie ein mit der Diaphyse 72 sich lang erstreckendes Langloch 61 in dem Flachsteg 41. Die Verankerung kommt in einem relativ festen Bereich des Humerus 1 an der Diaphyse 72 zu liegen, und sie trägt am Umfang der Diaphyse 72 mit dem besonders flach und kleinflächig ausgebildeten Flachsteg 41 des Diaphyseankers 4 nicht besonders auf. Vorteilhaft kann der Befestigungsteil des Flachstegs 41 derart elastisch und damit so dünn ausgebildet werden, dass er sich nach Anbringen des Verbindungsmittels 5 und des Verankerungsmittels 6 an die Diaphysewandung anschmiegt. Die Verbindung des distalen Stützkörperendes mit der Verankerungsschraube 62 oder einem anderen geeigneten Verbindungsmittel auch über den zur elastischen Anformung elastischen Steg 41 erweist sich für die erfindungsgemäße Stabilisierung als ausreichend wirksam.

Wie insbesondere aus Fig. 1 ersichtlich, ist das Langloch 61 am Innenrand mit drei Rastlöchern 611 geformt, die bei angezogener Schraubverbindung jeweils den Kopf der Verankerungsschraube 62 in festem Rastsitz aufnehmen, während bei nicht angezogener Schraubverbindung über die Länge des Langlochs 61 ein Durchgang für den Schaft der Verankerungsschraube 62 ausgebildet ist. Mit dieser Einrichtung lässt sich der Diaphyseanker 4 wahlweise in drei seine Höhenlage an der Diaphyse 72 bestimmende Rastpositionen festsetzen.

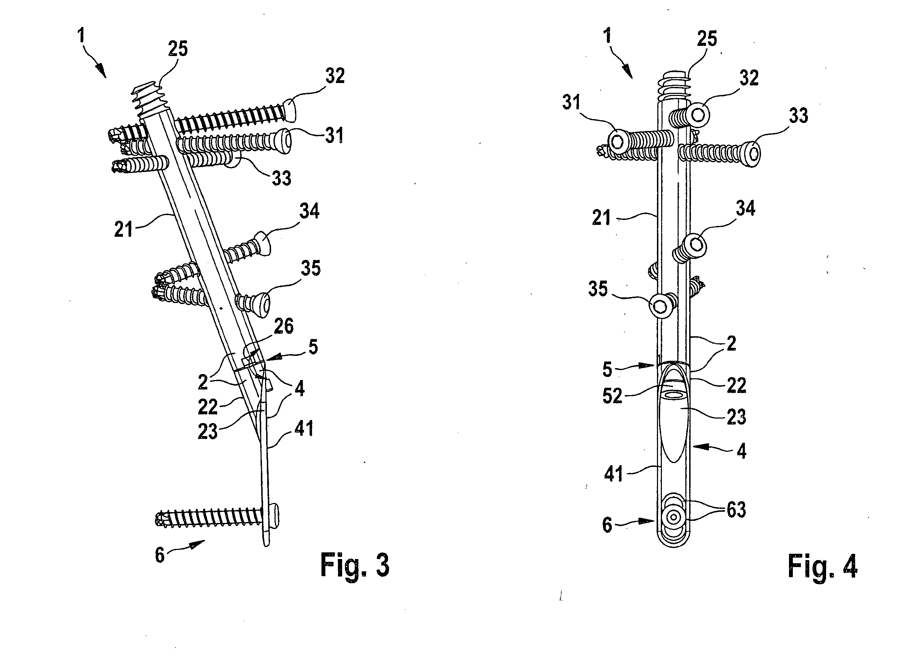

Im Ausführungsbeispiel der Fig. 2 bis 4 sitzt der Schraubenkopf in dem mittleren Rastsitz. Jeder Rastsitz sichert den festgesetzten Diaphyseanker 4 auf einfache Weise gegen von der Epiphyse 71 längs der Diaphyse 72 weg gerichtete Bewegung. Man erhält in gewünschter Höhenposition eine besonders wirksame Abstützung in Richtung quer zum Schaft der Verankerungsschraube 62. Andere geeignete, die Stützung in der Höhe einstellbare Rastverbindungen od. dgl. können eingerichtet werden. Dabei werden die Verbindungs-/Verankerungselemente so ausgebildet und dimensioniert, dass die Einrichtung an der Diaphyse 72 entsprechend der flachen Dimension des Flachstegs 41 besonders flach bleibt.

Die Befestigungsstifte 3, wie aus Fig. 1 bis 4 ersichtlich, sind als Schrauben mit über ihre Länge durchgehendem Gewinde (Vollgewinde), zweckmäßig als kanülierte Lochschrauben ausgeführt. Diese werden durch jeweils ein Innengewinde aufweisende Querdurchbohrungen 24 gesetzt, die ausschließlich an dem Hauptkörper 21 des Stützkörpers 2 angebracht sind, und zwar so, dass die Schrauben 3 in kreuzender Anordnung zu liegen kommen. Jede Schraube (jeder Befestigungsstift) 3 wird mittels der Schraubverbindung winkelfest an dem Stützkörper 2 und mit Sicherung gegen Bewegung längs der Schraubenachse angebracht.

Wie aus Fig. 2 ersichtlich, kommt der Kurznagel 1 bzw. der Stützkörper 2 in besonderer Schräganordnung im Wesentlichen im Humeruskopf (Frakturepiphyse) 71 zu liegen. Der Stützkörper 2 ist so dimensioniert und angeordnet, dass er sich, ausgehend von seinem distalen Ende im Bereich des chirurgischen Halses des Knochens in den medialen Bereich des Humeruskopfes 71 hinein erstreckt. Das proximale Ende des Stützkörpers 2 ist mit einem Außengewinde 25 versehen. Dieses Gewinde 25 ist selbstschneidend und verankert den Stützkörper 2 an seinem knochenseitigen Eingriffsende. Diese Verankerung wirkt mit der distalen lateralen Verankerung mittels des erfindungsgemäßen Diaphyseankers 4 besonders gut zusammen.

Das Implantat wird dadurch hergestellt, dass der Kurznagel 1 unter Verwendung von üblichen Instrumenten und Werkzeugen wie insbesondere Zielgerät, Bohrwerkzeugen, Führungsspießen und - hülsen implantiert wird. Zunächst wird der Hauptkörper 21 auf lateralem, gelenkfernem Zugangsweg 70 mit sattem Sitz in ein zuvor gefertigtes Bohrloch 701 eingebracht, das zur Aufnahme des Stützkörpers 2 dient und über dessen Gesamtlänge mit gleichem Querschnitt einheitlich ist. Dann kann zum Beispiel die Schraube 31 zur Fixierung des Tuberculum minus eingebracht werden. So dann werden die Schrauben 32 und 33 zum Fixieren des Tuberculum majus eingebracht. Diese Schrauben, die von dem Stützkörper 2 getragen werden, verbinden jeweils Fragmente nach Reposition. Sie durchsetzen jeweils eine (nicht dargestellte) Endstellen-Bruchfläche und halten die Fragmente durch Schraubverbindung aneinander. Schließlich wird der Diaphyseanker 4 mit dem Stützkörperfuß 22 mittels des Verbindungsmittels 5 angefügt. Die Verankerung mit dem Mittel 6 erfolgt distal, fern von dem an der Epiphyse 71 tiefsten Bruchausläufer.

Im Ausführungsbeispiel weist der Kurznagel 1 noch Befestigungsschrauben (Befestigungsstifte) 34, 35 auf, die im Bereich des chirurgischen Halses bzw. im Bereich der Metaphyse eingeschraubt werden und dazu dienen, den Kurznagel 2 statt der Verankerung mit proximalem Gewinde 25 oder zusätzlich in seinem Längsbereich in einem festeren Knochenbereich zu stabilisieren. Von wesentlicher Bedeutung bleibt auch insoweit das versenkte Anbringen des erfindungsgemäßen Diaphyseankers 4 am äußersten distalen Ende des Stütznagels 2 mit Verankerung fern von dem Frakturbereich in festem Knochenmaterial der geraden Diaphyse 72.

## Patentansprüche

1. Kurznagel (1) zum Versorgen von gelenknahen Epihysefrakturen eines Epiphyse (71) und Diaphyse (72) aufweisenden Röhrenknochens (7), umfassend einen an die Dimension der Frakturepiphyse (71) angepassten, zum Einbringen mit lateralem, gelenkfernem Zugangsweg (70) eingerichteten, zu implantierenden Nagelstützkörper (2) und, im Zustand der Frakturversorgung, wenigstens einen den Nagelstützkörper (2) mit diesem in gekreuzter Anordnung durch Querdurchbohrung (24) durchsetzenden BefestigungsStift (3), der mit winkelfester Anbringung an dem Nagelstützkörper (2) und mit Sicherung gegen Bewegung längs der Stiftachse vorgesehen ist, wobei der Nagelstützkörper (2) so dimensioniert und eingerichtet ist, dass er in der Frakturepiphyse (71) in Schräglage zu liegen kommt und sein an der lateralen Einbringungsseite zu liegen kommendes Ende eine Diaphyseschräge (23) aufweist, die am Außenumfang der Diaphyse (72) in einem Diaphysebereich wenigstens nahezu bündig abschließt, der an die sich zwischen der Frakturepiphyse (71) und der Diaphyse (72) erstreckende Metaphyse (73) anschließt, **dadurch gekennzeichnet, dass** der Nagelstützkörper (2) zweiteilig mit einem die wenigstens eine Querdurchbohrung für den Befestigungsstift (3) aufweisenden Hauptkörper (21) und einem die Diaphyseschräge (23) aufweisenden Stummelkörper (22) ausgebildet ist, der mit dem Hauptkörper (21) entsprechendem Stützquerschnitt versehen und fester Bestandteil eines Diaphyseankers (4) ist, der mittels des Stummelkörpers (22) und eines den Hauptkörper (21) und den Stummelkörper (22) steif verbindenden Verbindungsmittels (5) an den Hauptkörper (21) lösbar ansetzbar ist und der mit einem Verbindungs-Flachsteg (41) ausgebildet ist, der in schräger Ausrichtung mit der Diaphyseschräge (23) mit dieser lateral flächig abschließt, von der Frakturepiphyse (71) wegweisend flach an der Diaphyse (72) zur Anlage kommt und mit einem Verankerungsmittel (6) ausgestattet ist, durch das der Flachsteg (41) in flacher Anlage an der Diaphyse (72) festsetzbar ist.

2. Kurznagel nach Anspruch 1, **dadurch gekennzeichnet, dass** der Nagelstützkörper (2) an seinem dem Stummelkörper (22) abgewandten Ende mit einem Außengewinde (25) versehen ist.

3. Kurznagel nach Anspruch 1 oder 2, **dadurch gekennzeichnet, dass** das den Hauptkörper (21) und den Stummelkörper (22) des Stützkörpers (2) verbindende Verbindungsmittel (5) durch Schraubverbindung (51) gebildet ist.

4. Kurznagel nach Anspruch 3, **dadurch gekennzeichnet, dass** der Stummelkörper (22) mit Hohlraum (220) ausgebildet ist und die Schraubverbindung (5) eine von lateral setzbare Verbindungsschraube (52) mit einem Kopfbereich umfasst, der in dem Stummelkörper-Hohlraum (220) zumindest im Wesentlichen versenkt zu liegen kommt.

5. Kurznagel nach einem der Ansprüche 1 bis 4, **dadurch gekennzeichn e t,** dass das den Hauptkörper (21) und den Stummelkörper (22) des Stützkörpers (2) verbindende Verbindungsmittel (5) mit einer Drehsicherung (53) eingerichtet ist, die eine relative axiale Drehung zwischen den beiden Körpern (21, 22) in einstellbarer Drehposition sperrt.

6. Kurznagel einem der Ansprüche 1 bis 5, **dadurch gekennzeichnet, dass** das Verankerungsmittel (6) des Diaphyseankers (4) ein entsprechend der Diaphyse (72) sich erstreckendes, an dem Verbindungs-Flachsteg (41) ausgebildetes Langloch (61) und eine durch dieses hindurchsetzbare Verankerungsschraube (62) umfasst.

7. Kurznagel nach einem der Ansprüche 1 bis 6, **dadurch gekennzeichnet, dass** das Verankerungsmittel (6) des Diaphyseankers (4) mit einem Rastmittel (63) eingerichtet ist, das wenigstens zwei jeweils eine Höhenlage des Diaphyseankers (4) an der Diaphyse (72) bestimmende Rastpositionen ausbildet, die wählbar sind und den Diaphyseanker (4) in festgesetztem Zustand gegen von der Epiphyse (71) längs der Diaphyse (72) weg gerichtete Bewegung in gewählter Rastposition sichern.

8. Kurznagel nach einem der Ansprüche 1 bis 7, **dadurch gekennzeichnet, dass** der Stummelkörper (22) des Stützkörpers (21) und der Verbindungs-Flachsteg (41) so dimensioniert und geformt sind, dass der Verbindungs-Flachsteg (41) zur Seite der Metaphyse (73) mit dem Stummelkörper (22) zumindest im Wesentlichen überstandsfrei abschließt.

9. Stützkörper (2) eines Kurznagels (1) nach einem der Ansprüche 1 bis 8, wobei der Stützkörper (2) mit dem Hauptkörper (21) und dem einen Bestandteil des Diaphyseankers (4) bildenden Stummelkörper (22) zweiteilig ausgebildet ist.

10. Sachgesamtheit zum Herstellen eines Kurznagel-Implantates, insbesondere für proximale Humerusfrakturen, umfassend die Teile des Kurznagels (1) bzw. des Stützkörpers (2) nach einem der Ansprüche 1 bis 9, nämlich wenigstens in Einzahl jeweils den Hauptkörper (21), den Diaphyseanker (4) mit Stummelkörper (22), dafür das Verbindungsmittel (5) und das Verankerungsmittel (6) sowie wenigstens einen Satz von Befestigungsstiften (3).

## Claims

1. Short nail (1) for the treatment of epiphyseal fractures close to the joint in a long bone (7) having epiphysis (71) and diaphysis (72), comprising a nail supporting body (2) to be implanted, adapted to the dimensions of the fractured epiphysis (71) and designed to be introduced with a lateral access path (70) remote from the joint, and, in the state of treatment of the fracture, at least one fixation pin (3) which passes through the nail supporting body (2) in a crossed arrangement with the latter through a transverse through-bore (24) and which is provided with mounting at a fixed angle on the nail supporting body (2) and with protection against movement along the axis of the pin, wherein the nail supporting body (2) is dimensioned and designed in such a way that it comes to lie in an oblique position in the fractured epiphysis (71), and its end which comes to lie on the lateral side of introduction has a diaphyseal slant (23) which ends at least almost flush at the outer circumference of the diaphysis (72) in a region of the diaphysis which adjoins the metaphysis (73) extending between the fractured epiphysis (71) and the diaphysis (72), **characterised in that** the nail supporting body (2) is constructed in two parts with a main body (21) having the at least one transverse through-bore for the fixation pin (3) and a stub body (22) which has the diaphyseal slant (23) and which is provided with a supporting cross-section corresponding to the main body (21) and forms a fixed part of a diaphyseal anchor (4) which can be releasably attached to the main body (21) by means of the stub body (22) and a connecting means (5) rigidly connecting the main body (21) and the stub body (22) and which is designed with a flat connecting web (41) which in oblique orientation ends laterally in a plane with the diaphyseal slant (23), comes to abut flat against the diaphysis (72), pointing away from the fractured epiphysis (71), and is provided with an anchoring means (6) by which the flat web (41) can be fixed in flat abutment against the diaphysis (72).

2. Short nail according to claim 1, **characterised in that** the nail supporting body (2) at its end facing away from the stub body (22) is provided with an external thread (25).

3. Short nail according to claim 1 or 2, **characterised in that** the connecting means (5) which connects the main body (21) and the stub body (22) of the supporting body (2) consists of a screw connection (51).

4. Short nail according to claim 3, **characterised in that** the stub body (22) is provided with a cavity (220) and the screw connection (5) comprises a laterally insertable connecting screw (52) with a head region which comes to lie at least substantially countersunk in the stub body cavity (220).

5. Short nail according to any one of claims 1 to 4, **characterised in that** the connecting means (5) which connects the main body (21) and the stub body (22) of the supporting body (2) is designed with means (53) for protection against rotation, which blocks relative axial rotation between the two bodies (21, 22) in an adjustable rotational position.

6. Short nail according to any one of claims 1 to 5, **characterised in that** the anchoring means (6) of the diaphyseal anchor (4) comprises an elongated hole (61) extending according to the diaphysis (72) and formed in the flat connecting web (41), and an anchoring screw (62) which can be passed through the elongated hole.

7. Short nail according to any one of claims 1 to 6, **characterised in that** the anchoring means (6) of the diaphyseal anchor (4) is designed with a catch means (63) which forms at least two catch positions which in each case determine a height of the diaphyseal anchor (4) on the diaphysis (72) and which can be selected and in the selected catch position protect the diaphyseal anchor (4) in the fixed state against movement directed away from the epiphysis (71) along the diaphysis (72).

8. Short nail according to any one of claims 1 to 7, **characterised in that** the stub body (22) of the supporting body (21) and the flat connecting web (41) are dimensioned and shaped in such a way that the flat connecting web (41) ends at least substantially without overhang with the stub body (22) on the side of the metaphysis (73).

9. Supporting body (2) of a short nail (1) according to any one of claims 1 to 8, wherein the supporting body (2) is constructed in two parts with the main body (21) and the stub body (22) which forms part of the diaphyseal anchor (4).

10. Entity of items for producing a short nail implant, in particular for proximal fractures of the humerus, comprising the parts of the short nail (1) or supporting body (2), according to any one of claims 1 to 9, namely at least in the singular in each case the main body (21), the diaphyseal anchor (4) with stub body (22), the connecting means (5) and the anchoring means (6) therefor, and also at least one set of fixation pins (3).

## Revendications

1. Clou court (1) destiné au traitement de fractures de l'épiphyse, proches d'une articulation, d'un os long (7) comportant une épiphyse (71) et une diaphyse (72), ce clou comprenant un corps (2) de soutien du clou à implanter, adapté à la dimension de l'épiphyse fracturée (71), configuré de façon à être introduit par une voie (70) d'accès latérale éloignée de l'articulation, et, à l'état de traitement de la fracture, au moins une tige (3) de fixation traversant le corps (2) de soutien du clou en disposition croisée avec celui-ci par un trou traversant transversal (24), tige qui est prévue de façon à être rapportée, selon un angle fixe, au corps (2) de soutien du clou, et avec une sécurité l'empêchant de se déplacer le long de l'axe de la tige, le corps (2) de soutien du clou étant dimensionné et configuré de telle sorte qu'il vient se loger dans l'épiphyse fracturée (71) en position inclinée et que son extrémité venant se loger sur le côté latéral d'introduction présente un plan incliné (23) de la diaphyse, qui se termine, au moins presque en affleurement, au niveau de la circonférence extérieure de la diaphyse (72) en une zone de diaphyse qui est raccordée à la métaphyse (73) s'étendant entre l'épiphyse fracturée (71) et la diaphyse (72) ; clou court **caractérisé en ce que** le corps (2) de soutien du clou est configuré en deux parties, avec un corps principal (21) comprenant au moins un trou traversant transversal pour la tige de fixation (3) et un corps (22) formant moignon comportant le plan incliné (23) de la diaphyse ; le corps formant moignon est doté d'une section de soutien correspondant au corps principal (21) et est un constituant fixe d'une ancre (4) pour diaphyse, laquelle peut être appliquée d'une manière amovible sur le corps principal (21) à l'aide du corps formant moignon (22) et d'un moyen (5) de liaison reliant de manière fixe le corps principal (21) et le corps formant moignon (22), et qui est formée par une traverse plate (41) de liaison, qui se termine, obliquement par rapport au plan incliné (23) de la diaphyse, en épousant la surface latérale de ce dernier, qui vient s'appuyer sur la diaphyse (72) à plat, en s'écartant de l'épiphyse fracturée (71), et qui est pourvue d'un moyen (6) d'ancrage, par lequel la traverse plate (41) peut être fixée à plat contre la diaphyse (72).

2. Clou court selon la revendication 1 **caractérisé en ce que** le corps (2) de soutien du clou est doté d'un filetage extérieur (25) sur son extrémité opposée au corps formant moignon (22).

3. Clou court selon les revendications 1 ou 2 **caractérisé en ce que** le moyen (5) de liaison reliant le corps principal (21) et le corps formant moignon (22) du corps (2) de soutien est formé par une liaison à vis (51).

4. Clou court selon la revendication 3 **caractérisé en ce que** le corps formant moignon (22) est configuré avec une cavité (220) et **en ce que** la liaison à vis (5) comprend une vis (52) de liaison pouvant être placée par le côté et ayant une zone de tête qui vient se loger dans la cavité (220) du corps formant moignon en étant au moins sensiblement enfoncée.

5. Clou court selon l'une quelconque des revendications 1 à 4 **caractérisé en ce que** le moyen (5) de liaison reliant le corps principal (21) et le corps formant moignon (22) du corps (2) de soutien est configuré avec une sécurité (53) contre la rotation, qui bloque une rotation axiale relative entre les deux corps (21, 22) dans une position de rotation réglable.

6. Clou court selon l'une quelconque des revendications 1 à 5 **caractérisé en ce que** le moyen (6) d'ancrage de l'ancre (4) pour diaphyse comprend un trou longitudinal (61) formé sur la traverse plate (41) de liaison, s'étendant suivant la diaphyse (72) et une vis (62) d'ancrage pouvant être introduite à travers celui-ci.

7. Clou court selon l'une quelconque des revendications 1 à 6 **caractérisé en ce que** le moyen (6) d'ancrage de l'ancre (4) pour diaphyse est configuré avec un moyen (63) d'arrêt, qui forme au moins deux positions d'arrêt définissant chacune une position en hauteur de l'ancre (4) pour diaphyse sur la diaphyse (72), ces positions étant sélectionnables et immobilisant l'ancre (4) de diaphyse dans la position d'arrêt choisie à l'état fixe contre un déplacement en sens opposé à l'épiphyse (71) le long de la diaphyse (72).

8. Clou court selon l'une quelconque des revendications 1 à 7 **caractérisé en ce que** le corps formant moignon (22) du corps (21) de soutien et la traverse plate (41) de liaison sont dimensionnés et formés de telle sorte que la traverse plate (41) de liaison se termine au moins sensiblement sans saillie par le corps formant moignon (22) du côté de la métaphyse (73).

9. Corps (2) de soutien d'un clou court (1) selon l'une quelconque des revendications 1 à 8, le corps (2) de soutien étant configuré en deux parties avec le corps principal (21) et le corps formant moignon (22), qui forme une partie de l'ancre (4) de diaphyse.

10. Ensemble destiné à la fabrication d'un clou court à implanter, notamment pour des fractures proximales de l'humérus, comprenant les parties du clou court (1) ou du corps (2) de soutien selon l'une quelconque des revendications 1 à 9, c'est-à-dire, au moins à l'unité, le corps principal (21), l'ancre (4) pour diaphyse avec le corps formant moignon (22), et pour cela le moyen (5) de liaison et le moyen (6) d'ancrage ainsi qu'au moins un jeu de tiges (3) de fixation.
